# EUROPÄISCHE PATENTSCHRIFT

(11) **EP 2 262 760 B1**
(45) Veröffentlichungstag und Bekanntmachung des Hinweises auf die Patenterteilung: **30.08.2017**
(21) Anmeldenummer: 09716225.9
(22) Anmeldetag: 02.03.2009
(51) Int. Cl.: C07C 227/18

(54) **VERFAHREN ZUR HERSTELLUNG VON AMINODICARBONSÄURE-N,N-DIESSIGSÄUREN**
METHOD FOR THE PRODUCTION OF AMINODICARBOXYLIC ACID-N,N-DIACETIC ACIDS
PROCEDE DE PREPARATION D'ACIDES N,N-DIACETIQUES D'ACIDE AMINODICARBOXYLIQUE

(30) Priorität: 03.03.2008 EP 08152207
(43) Veröffentlichungstag der Anmeldung: 22.12.2010
(73) Patentinhaber: BASF SE, 67056 Ludwigshafen am Rhein (DE)
(72) Erfinder: OFTRING, Alfred, 67098 Bad Dürkheim (DE); STAMM, Armin, 55268 Nieder-Olm (DE); WIRSING, Friedrich, 67567 Kaiserslautern (DE); BRAUN, Gerold, 67071 Ludwigshafen (DE)
(74) Vertreter: BASF IP Association
(86) Internationale Anmeldenummer: PCT/EP2009/052447
(87) Internationale Veröffentlichungsnummer: WO 2009/109544

(56) Entgegenhaltungen:
- EP-A- 0 884 381
- WO-A-2009/024518
- DE-A1- 2 339 888
- DE-A1- 4 211 713

## Beschreibung

Die vorliegende Erfindung betrifft ein Verfahren zur Herstellung von Aminodicarbonsäure-N,N-diessigsäuren der allgemeinen Formel I 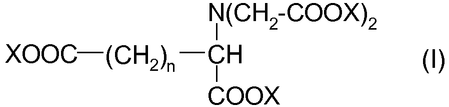 worin X unabhängig voneinander für Wasserstoff oder Alkalimetall steht und n eine Zahl 1 oder 2 bezeichnet. Weiterhin betrifft die Erfindung Aminodicarbonsäure-N,N-diessigsäuren hoher Reinheit.

Aminodicarbonsäure-N,N-diessigsäuren I sind vor allem als Komplexbildner in Wasch- und Reinigungsmitteln von Interesse. Die für diese Zwecke gebräuchlichen Stoffe wie Polycarboxylate, Phosphonate, Triphosphate, Ethylendiamintetraessigsäure (EDTA) und Nitrilotriessigsäure (NTA) sind mit Nachteilen behaftet. Insbesondere tragen sie zur Eutrophierung bei, sind biologisch schwer abbaubar oder haben toxische Wirkungen. Eine Alternative stellt die preisgünstige und biologisch abbaubare Glutaminsäure-N,N-diessigsäure (GLDA) bzw. ihre Salze dar, die sich mit n = 2 aus Formel I ergibt.

Verfahren zur Herstellung der N,N-Diessigsäurederivate von α-Aminosäuren sind grundsätzlich seit langem bekannt. So beschreibt US 2500019 die Herstellung derartiger Diessigsäurederivate durch Umsetzung von α-Aminosäuren mit Formaldehyd und Natriumcyanid vorzugsweise in stark basischer wässriger Lösung bei Temperaturen von 30 bis 100°C. Mit Glutaminsäure als α-Aminosäure wurde eine Mischung aus Glutaminsäure-N,N-Diessigsäure und α-Aminobuttersäure-N,N-Diessigsäure erhalten, da unter den stark basischen Bedingungen die endständigen Carboxylgruppe teilweise decarboxyliert.

In DE 4211713 werden Verfahren zur Herstellung von Aminodicarbonsäure-N,N-diessigsäuren auf Basis von sauren und basischen Strecker-Reaktionen beschrieben. Hierbei setzt man die α-Aminosäure mit wenigstens 2 mol Formaldehyd und wenigstens 2 mol Blausäure oder Alkalimetallcyanid um. Die vergleichsweise niedrige Ausbeute von 91% bezüglich der Asparaginsäure lässt vermuten, dass unerwünschte Nebenprodukte, wie etwa NTA, entstanden sind, die schwer abtrennbar sind.

Der vorliegenden Erfindung liegt somit die Aufgabe zu Grunde, ein einfach durchzuführendes Verfahren zur Herstellung von Aminodicarbonsäure-N,N-diessigsäuren der oben bezeichneten allgemeinen Formel I bereitzustellen, das diese Verbindungen in guten Ausbeuten mit niedrigen Nebenproduktanteilen liefert. Insbesondere soll der Anteil an NTA möglichst klein sein. Eine weitere Aufgabe der vorliegenden Erfindung ist die Bereitstellung solcher Aminodicarbonsäure-N,N-diessigsäuren hoher Reinheit.

Diese und weitere Aufgaben werden durch das im Folgenden näher beschriebene Verfahren gelöst.
Gegenstand der vorliegenden Erfindung ist ein Verfahren zur Herstellung von Aminodicarbonsäure-N,N-diessigsäuren der oben bezeichneten allgemeinen Formel I, umfassend die folgenden Schritte:
a) Umsetzung einer Aminodicarbonsäure der allgemeinen Formel II 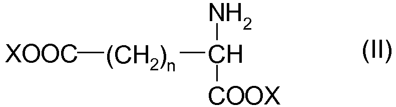 worin X und n die zuvor genannten Bedeutungen haben, mit 0,8 bis 1,2 Mol-Äquivalenten Formaldehyd und mit 0,8 bis 1,2 Mol-Äquivalenten Blausäure;
   umfassend die Zugabe von Formaldehyd und Blausäure, wobei man die Aminodicarbonsäure im Reaktionsgefäß vorlegt und Formaldehyd und Blausäure zugibt;
   Nachreagieren lassen über einen Zeitraum von einer Minute bis 5 Stunden,
b) anschließende Umsetzung des in Schritt a) erhaltenen Reaktionsprodukts mit 0,8 bis 1,2 Mol-Äquivalenten Blausäure und mit 0,8 bis1,2 Mol-Äquivalenten Formaldehyd;
c) Hydrolyse des in Schritt b) erhaltenen Reaktionsprodukts.

Das erfindungsgemäße Verfahren ist mit einer Reihe von Vorteilen verbunden. Zum einen ist es vergleichsweise einfach beispielsweise in einer Eintopf-Synthese mit kurzen Zykluszeiten durchzuführen. Zudem liefert es die gewünschten Aminodicarbonsäure-N,N-diessigsäuren in guten Ausbeuten und hoher Reinheit, so dass aufwendige Reinigungsverfahren nicht nötig sind. Das Verfahren lässt sich außerdem auch in industriellem Maßstab gut handhaben.

Im Rahmen des erfindungsgemäßen Verfahrens wird unter Formaldehyd der Reaktand Formaldehyd, entweder im gasförmigen Zustand oder gelöst in einem wässrigen Lösungsmittel, vorzugsweise in einer Konzentration von 20 bis 50 Gew.-%, oder Formaldehyd-Äquivalente verstanden. Solche Äquivalente sind Verbindungen, die Formaldehyd im Reaktionsverlauf freisetzen oder direkt zu denselben Produkten wie Formaldehyd umgesetzt werden. Dem Fachmann bekannte Beispiele für solche Äquivalente sind Paraformaldehyd, Trioxan und Methylal sowie Mischungen hieraus oder Mischungen mit Formaldehyd. Bevorzugt wird eine wässrige Lösung von Formaldehyd eingesetzt.

Im Rahmen des erfindungsgemäßen Verfahrens wird der Reaktand Blausäure entweder in wässriger Lösung oder vorzugsweise in reiner Form eingesetzt.

In Schritt a) des erfindungsgemäßen Verfahrens wird die freie Aminodicarbonsäure II oder die teilweise oder vollständig neutralisierte Aminodicarbonsäure II eingesetzt, wobei eine Neutralisation vorzugsweise mit Alkalimetallbasen wie Natriumhydroxid, Kaliumhydroxid, Natriumcarbonat, Kaliumcarbonat, Natriumhydrogencarbonat oder Kaliumhydrogencarbonat, erfolgt. Es können auch Mono- oder Dialkalimetallsalze von II oder Gemische davon eingesetzt werden, wobei die Natriumsalze bevorzugt sind. Bezogen auf die eingesetzte Verbindung II setzt man in der Regel erfindungsgemäß 0,8 bis 1,2 Mol-Äquivalente, insbesondere 0,9 bis 1,1 Mol-Äquivalente Formaldehyd und 0,8 bis 1,2 Mol-Äquivalente, insbesondere 0,9 bis 1,1 Mol-Äquivalente, Blausäure ein.

Im Falle der Glutaminsäure wird vorzugsweise ihr Monoalkalimetallsalz, insbesondere das Mononatriumsalz-Monohydrat, eingesetzt. Hingegen wird die Asparaginsäure vorzugsweise in Form der freien Aminodicarbonsäure eingesetzt. Unter den Aminodicarbonsäuren der Formel II sind die L-Isomere oder ihre Salze bevorzugt.

Die Umsetzung in Schritt a) erfolgt in der Regel in einem Lösungsmittel. Die Konzentration der Aminodicarbonsäure bzw. ihres Salzes beträgt dabei vorzugsweise 10 bis 60 Gew.-% und insbesondere 20 bis 50 Gew.-%, bezogen auf das Gesamtgewicht des Reaktionsgemisches.

In einer bevorzugten Ausführungsform erfolgt die Umsetzung in einem wässrigen Lösungsmittel. Im Rahmen des erfindungsgemäßen Verfahrens wird unter wässrigem Lösungsmittel Wasser oder Mischungen aus Wasser mit wasserlöslichen organischen Lösungsmitteln verstanden. Als organische Lösungsmittel kommen hier insbesondere Methanol, Ethanol, n-Propanol, iso-Propanol, tertiär-Butanol, Dioxan oder Tetrahydrofuran oder Mischungen dieser organischen Lösungsmittel untereinander in Frage. Der Anteil an wasserlöslichen organischen Lösungsmittel wird vorzugsweise 50 Vol.-% und insbesondere 20 Vol.-%, bezogen auf die Gesamtmenge des wässrigen Lösungsmittels, nicht überschreiten.

Vorzugsweise wird die Aminodicarbonsäure II oder ein Salz von II im Reaktionsgefäß vorgelegt, vorzugsweise in dem gewünschten Lösungsmittel.

Die Reaktanden Formaldehyd und Blausäure können gleichzeitig oder nacheinander zugegeben werden. In einer bevorzugten Ausführungsform wird zunächst das Formaldehyd und anschließend die Blausäure zu der Aminodicarbonsäure der Formel II bzw. ihrem Salz gegeben. Hierunter versteht man, dass die Hauptmenge, insbesondere wenigstens 80 % des Formaldehyds in das Reaktionsgefäß gegeben werden, bevor die Zugabe der Blausäure erfolgt. Es ist auch bevorzugt, die beiden Reaktanden jeweils über relative kurze Zeiträume von etwa 10 Sekunden bis zu 1 Stunde, insbesondere in Abhängigkeit von insbesondere der Temperaturentwicklung oder anderen Faktoren wie beispielweise der Ansatzgröße, zuzugeben. Nach beendeter Zugabe lässt man in der Regel nachreagieren, zum Beispiel über einen Zeitraum von etwa 1 Minute bis zu 5 Stunden, insbesondere so lange, bis keine Exothermie mehr feststellbar ist.

Die Umsetzungen des Schrittes a) erfolgen vorzugsweise unter Temperaturkontrolle und üblicherweise bei Temperaturen im Bereich von 0 bis 100°C, insbesondere von 10 bis 70°C. Entsprechend einer bevorzugten Ausführungsform wird während der Umsetzungen weder extern erhitzt noch extern gekühlt.

Man nimmt an, dass in Schritt a) als Hauptkomponente das Aminodicarbonsäure-N-monoacetonitril gebildet wird. Als Nebenkomponenten können die als Edukt eingesetzte Aminodicarbonsäure, das Aminodicarbonsäure-N,N-diacetonitril, sowie Hydrolyseprodukte der genannten Acetonitrilverbindungen, beispielsweise die entsprechenden Amide und partiellen Amide, als auch die Salze der vorgenannten Verbindungen auftreten.

Das Reaktionsprodukt des Schrittes a) kann entweder mit oder ohne vorherige Aufarbeitung im Schritt b) des erfindungsgemäßen Verfahrens eingesetzt werden. Zur Aufarbeitung können dem Fachmann vertraute Verfahren zur Entfernung des Lösungsmittels und gegebenenfalls zur Aufreinigung des Produkts, wie beispielweise Einengen zur Trockne, Sprüh- oder Gefriertrocknung, Fällung und Kristallisation, verwendet werden. Vorzugweise wird aber keine Aufarbeitung durchgeführt und die sich anschließende Umsetzungen gemäß des Schrittes b) erfolgen direkt in der im Schritt a) erhaltenen Reaktionsmischung.

In Schritt b) wird das aus Schritt a) erhaltene Reaktionsprodukt mit, bezogen auf die eingesetzte Verbindung der Formel II, 0,8 bis1,2 Mol-Äquivalenten, insbesondere 0,9 bis 1,1 Mol-Äquivalenten, Blausäure und mit 0,8 bis 1,2 Mol-Äquivalenten, insbesondere 0,9 bis 1,1 Mol-Äquivalenten Formaldehyd, umgesetzt.

Die Umsetzung in Schritt b) erfolgt typischerweise durch Zugabe der Bausäure und des Formaldehyds zu dem in Schritt a) erhaltenen Reaktionsprodukts. In einer bevorzugten Ausführungsform erfolgt die Zugabe der Reaktanden Blausäure und Formaldehyd direkt in die Reaktionsmischung, die in Schritt a) erhalten wurde. Falls im Anschluss an Schritt a) das Lösungsmittel teilweise oder vollständig entfernt worden ist, wird vorzugsweise zuvor in einem wässrigen Lösungsmittel aufgenommen.

Die Reaktanden Blausäure und Formaldehyd können in Schritt b) gleichzeitig oder nacheinander zu der in Schritt a) erhaltenen Reaktionsmischung gegeben werden. In einer bevorzugten Ausführungsform wird zunächst die Blausäure und anschließend das Formaldehyd zugegeben. Hierunter versteht man, dass die Hauptmenge, insbesondere wenigstens 80 % der Blausäure in das Reaktionsgefäß gegeben werden, bevor die Zugabe des Formaldehyds erfolgt.

In einer anderen Ausführungsform der Erfindung erfolgt in Schritt b) die Zugabe der Blausäure und des Formaldehyds parallel, d.h. die Hauptmenge, d.h. wenigstens 50 % und insbesondere wenigstens 80 % des Formaldehyds und der Blausäure werden innerhalb des gleichen Zeitraums zugegeben.

Es ist auch bevorzugt die beiden Reaktanden jeweils über relative kurze Zeiträume von etwa 10 Sekunden bis zu 1 Stunde, in Abhängigkeit insbesondere von der Temperaturentwicklung oder anderen Faktoren wie beispielweise der Ansatzgröße, zuzugeben.

Nach beendeter Zugabe lässt man in der Regel nachreagieren, zum Beispiel über einen Zeitraum von etwa 1 Minute bis zu 5 Stunden, insbesondere so lange, bis keine Exothermie mehr feststellbar ist.

Die Umsetzungen des Schrittes b) erfolgen vorzugsweise unter Temperaturkontrolle und üblicherweise bei Temperaturen von 0 bis 100°C, insbesondere von 20 bis 90°C. Entsprechend einer bevorzugten Ausführungsform wird während der Umsetzungen weder extern erhitzt noch extern gekühlt.

Für die Umsetzungen der Schritte a) und b) kommt generell ein weiter pH-Bereich in Betracht. Typischerweise führt man Umsetzungen mit Blausäure bei pH-Werten im Bereich von pH 0 bis pH 12, insbesondere von pH 1 bis pH 10, durch. Vorzugsweise lässt man die Reaktionen unter sich autogen einstellendem pH-Wert ablaufen und es werden keine Maßnahmen ergriffen, um den pH-Wert vor oder während den Umsetzungen zu justieren.

Das Reaktionsprodukt des Schrittes b) kann entweder mit oder ohne vorherige Aufarbeitung im Schritt c) des erfindungsgemäßen Verfahrens eingesetzt werden. Zur Aufarbeitung können dem Fachmann vertraute Verfahren zur Entfernung des Lösungsmittels und gegebenenfalls zur Aufreinigung des Produkts, wie beispielweise Einengen zur Trockne, Sprüh- oder Gefriertrocknung, Fällung und Kristallisation, verwendet werden. Vorzugweise wird aber keine Aufarbeitung durchgeführt, und die sich anschließende Umsetzungen gemäß des Schrittes c) erfolgen direkt mit der im Schritt b) erhaltenen Reaktionsmischung.

In Schritt c) wird das aus Schritt b) erhaltene Reaktionsprodukt sauer oder vorzugsweise basisch hydrolysiert, um vorliegende Nitrilgruppen und gegebenenfalls vorliegende Amidgruppen in Carboxylgruppen zu überführen. In der Regel wird Schritt c) unter Temperaturkontrolle und entsprechend bevorzugter Ausführungsformen entweder ohne oder, während des In-Kontakt-Bringens des Reaktionsprodukts aus Schritt b) mit der Base oder Säure, mit externer Kühlung durchgeführt.

Die saure Hydrolyse erfolgt vorzugsweise mit wässriger Salzsäure oder wässriger Schwefelsäure. Typischerweise gibt man die Säure, insbesondere in Abhängigkeit von der Temperaturentwicklung, innerhalb eines Zeitraums von 10 Minuten bis 10 Stunden und speziell von 30 Minuten bis 3 Stunden zu der in Schritt b) erhaltenen wässrigen Reaktionsmischung. Falls im Anschluss an Schritt b) das Lösungsmittel entfernt worden ist, wird in der Regel zuvor in einem wässrigen Lösungsmittel aufgenommen. Die Temperaturen während der Zugabe liegen üblicherweise bei 10 bis 100°C, insbesondere bei 20 bis 80°C.

Zur basischen Hydrolyse werden vorzugsweise wässrige Lösungen von Kaliumhydroxid oder insbesondere Natriumhydroxid, in Konzentrationen von 5 bis 50 Gew.-% und insbesondere von 20 bis 50 Gew.-%, eingesetzt. Typischerweise gibt man die in Schritt b) erhaltene wässrige Reaktionsmischung, insbesondere in Abhängigkeit von der Temperaturentwicklung, innerhalb eines Zeitraums von 10 Minuten bis 10 Stunden und speziell von 30 Minuten bis 3 Stunden direkt zu der Kalium- oder Natriumhydroxidlösung. Falls im Anschluss an Schritt b) das Lösungsmittel entfernt worden ist, wird in der Regel zuvor in einem wässrigen Lösungsmittel aufgenommen. Die Temperaturen während der Zugabe liegen üblicherweise bei 10 bis 100°C, insbesondere bei 20 bis 80°C.

Nach In-Kontakt-Bringen des Reaktionsprodukts aus Schritt b) mit der Base oder Säure wird in der Regel über einen Zeitraum von etwa 10 Minuten bis zu 10 Stunden zur Vervollständigung der Hydrolyse auf 60 bis 120°C, speziell auf 95 bis 110°C erhitzt, vorzugsweise bis die Ammoniakentwicklung beendet ist.

Die zur Herstellung des Tetranatriumsalzes der Aminodicarbonsäure-N,N-diessigsäure in der Hydrolyse üblicherweise einzusetzenden Mol-Äquivalente Natriumhydroxid, bezogen auf die in Schritt a) eingesetzte Verbindung II, lassen sich anhand folgender Formel ermitteln:

Mol-Äquivalente NaOH = ((4 bis 4,1) - Y)

wobei Y die Zahl der Carboxylatgruppen der Verbindung II ist. Geht man beispielsweise vom Mononatriumsalz der Aminodicarbonsäure aus, werden demnach 3,0 bis 3,1 Mol-Äquivalente Natriumhydroxid für die Hydrolyse benötigt. Analog kann das Mononatrium-Trikaliumsalz bei der Umsetzung mit in etwa der gleichen Anzahl an Mol-Äquivalenten Kaliumhydroxid erhalten werden. Die für die Herstellung von Verbindungen I mit anderen Kationenkombinationen notwendigen Bedingungen kann der Fachmann leicht ermitteln.

Alternativ können die Verbindungen II, vor ihrer Umsetzung in Schritt a), von 0 bis 100% im Falle der Monoalkalimetallsalze und von 0 bis 200% im Falle der freien Dicarbonsäuren teilweise oder vollständig neutralisiert werden. Eine solche Neutralisation erfolgt in der Regel mit Natrium- oder Kaliumbase. Die dann üblicherweise erforderlichen Mol-Äquivalente Alkalimetallhydroxid (MOH) für die basische Hydrolyse in Schritt c) errechnen sich wie folgt:

Mol-Äquivalente MOH = ((4 bis 4,1)- Y - Z/100)

wobei Y der obigen Definition entspricht und Z der Neutralisationsgrad im Bereich von 0 bis 100% für Y = 1, bzw. 0 bis 200% für Y = 0 ist. Wird beispielsweise das oben genannte Mononatriumsalz der Verbindung II zu 25% teilneutralisiert, ergibt sich anhand dieser Formel eine Menge von 2,75 bis 2,85 Mol-Äquivalenten MOH, die zur Herstellung des Tetraalkalimetallsalzes in Schritt c) einzusetzen wäre. Das gleiche Ergebnis erhält man für den Fall, dass die freie Dicarbonsäure der Verbindung II zu 125% teilneutralisiert eingesetzt wird.

Entsprechend einer weiteren Ausführungsform der Erfindung kann man das bei saurer Hydrolyse erhaltene Produkt mit einer Natrium- oder Kaliumbase wie beispielweise NaOH, KOH, Na₂CO₃, K₂CO₃, NaHCO₃ oder KHCO₃ neutralisieren und auf diese Weise das gewünschte Aminodicarbonsäure-N,N-diessigsäuresalz erhalten. Wie dem Fachmann ohne weiteres ersichtlich ist, lassen sich auch die Mono-, Di- und Trialkalimetallsalze durch den Einsatz geeigneter Mengen an Base herstellen. Des Weiteren lassen sich die verschiedenen gemischten Natrium-Kaliumsalze durch entsprechende Kombinationen von Natrium und Kaliumbasen erhalten.

Das erfindungsgemäße Verfahren kann sowohl in einem Batch-, einem Semibatch-, als auch in einem kontinuierlichen Verfahren durchgeführt werden. Bei kontinuierlicher Durchführung können in Schritt a) beispielsweise in einer ersten Reaktionszone parallel eine Verbindung II und Formaldehyd und anschließend in einer zweiten Reaktionszone Blausäure zudosiert werden. Schritt b) lässt sich beispielsweise in analoger Weise durchführen, wobei zunächst parallel das Reaktionsgemisch aus Schritt a) und die Blausäure und dann in einer weiteren Reaktionszone das Formaldehyd zugeführt wird. Alternativ können alle drei Komponenten dieses Schrittes parallel in einer Reaktionszone zusammengeführt werden. Zur basischen Hydrolyse kann beispielsweise das so erhaltene Reaktionsgemisch in einer weiteren Reaktionszone parallel mit Natriumhydroxidlösung dosiert werden. Hiervon ausgehend kann der Fachmann leicht entsprechende Semibatch-Verfahren entwickeln. Vorzugsweise wird für das erfindungsgemäße Verfahren ein Semibatch-Verfahren angewendet, bei dem die Schritte a) und b) in einem Batch-Reaktor, vorzugweise als Eintopf-Reaktion, und der Schritt c) vorzugsweise in einem 2. Batch-Reaktor mit Dosierung der Lösung b) zur vorgelegten Alkalilauge durchgeführt werden. Wegen weiterer Details wird auf das Beispiel der Anmeldung verwiesen.

Im Anschluss an die Hydrolyse kann die anfallende Reaktionsmischung direkt einer technischen Verwendung zugeführt werden. So stellt eine etwa 38 bis 40 Gew.-%ige wässrige Lösung des Tetranatriumsalzes der Verbindung I ein gängiges Verkaufsprodukt dar. Alternativ kann die Verbindung I aus der Reaktionsmischung der Hydrolyse isoliert und aufgearbeitet werden. Dazu bieten sich dem Fachmann bekannte Verfahren insbesondere zur Entfernung des Lösungsmittels und gegebenenfalls zur Aufreinigung an, wie beispielsweise Einengen zur Trockne, Sprüh- oder Gefriertrocknung, Fällung und Kristallisation. Typischerweise wird Verbindung I unmittelbar aus der Reaktionsmischung durch Entfernen des Lösungsmittels, vorzugsweise durch Sprühtrocknung, gewonnen.

Das erfindungsgemäße Verfahren liefert die Verbindungen der Formel I in hohen Ausbeuten von in der Regel über 88%, insbesondere über 92%, bezogen auf die eingesetzte Menge der Verbindung II. Durch das erfindungsgemäße Verfahren werden die Verbindungen der Formel I in hoher Reinheit gewonnen. Der NTA-Anteil liegt in der Regel unter 0,75 Gew.-% und insbesondere unter 0,25 Gew.-%, bezogen auf 100 % der Verbindung I.

Gegebenenfalls können sich weitere dem Fachmann bekannte Schritte zur Aufarbeitung und Konfektionierung anschließen, wie beispielsweise Fällung, Kristallisation und Umsalzung.

Auch mag sich ein Schritt zum Bleichen der wässrigen Lösung, beispielsweise durch Behandlung mit Aktivkohle oder durch Oxidation mittels Wasserstoffperoxid oder UV-Induktion, anbieten.

Weitere Gegenstände der Offenbarung sind die Bereitstellung von Salzen der Glutaminsäure-N,N-diessigsäure mit weniger als 0,75 Gew.-%, speziell mit weniger als 0,25 Gew.-% Salzen der NTA, bezogen auf 100 % der Verbindung I. Salze mit diesen Charakteristika lassen sich mit Hilfe der erfindungsgemäßen Verfahren bereitstellen.

Die folgenden Beispiele dienen der Erläuterung der Erfindung:

### Beispiel: Herstellung von L-Glutaminsäure-N,N-diessigsäure Tetranatriumsalz

In einem Reaktionsgefäß legte man eine ca. 40 Gew.-%ige Lösung von 187 g (1,0 mol) L-Glutaminsäure Mononatriumsalz Monohydrat in 280,5 g Wasser vor. Hierzu gab man unter Rühren und ohne Kühlung zunächst innerhalb von 30 Sekunden 100,0 g (1,0 mol) einer 30 Gew.-%igen wässrigen Formaldehyd-Lösung und anschließend innerhalb von 40 Sekunden 27,0 g (1,0 mol) Blausäure bei Umgebungstemperatur zu. Nach Beendigung der Zugabe rührte man weitere 5 Minuten. Danach war keine Wärmeentwicklung mehr feststellbar und die Temperatur lag bei 45°C. Die erhaltene Mischung wurde dann unter Rühren und ohne Kühlung zunächst innerhalb von 70 Sekunden mit 27,0 g (1,0 mol) Blausäure und anschließend innerhalb von 160 Sekunden mit 100,0 g (1,0 mol) einer 30 Gew.-%igen wässrigen Formaldehyd-Lösung versetzt, wobei die Temperatur auf etwa 60°C anstieg. Nach beendeter Zugabe rührte man noch etwa 5 Minuten. Die resultierenden etwa 720 g einer schwach gelblichen Lösung wurden dann innerhalb von 1 Stunde zu 489,6 g (3,06 mol) einer 25 Gew.-%igen Natronlauge bei 25°C dosiert. Nach Beendigung der Zugabe lag die Temperatur bei 60°C. Man erhitzte anschließend auf 100 bis 110°C bis nach etwa 4 Stunden keine Ammoniakentwicklung mehr feststellbar war. Es wurden 1100 g einer gelben Lösung erhalten, die laut HPLC-Analytik 330 g des L-Glutaminsäure-N,N-diessigsäure Tetranatriumsalzes enthielt und damit eine etwa 30 Gew.-%ige Lösung des Produkts darstellte. Bezogen auf eingesetztes Glutamat ergab sich eine Ausbeute von 94%. Der mittels HPLC bestimmte Anteil an NTA Trinatriumsalz in der Lösung betrug 0,05 Gew.-%.

### Vergleichsbeispiel:

Herstellung von L-Glutaminsäure-N,N-diessigsäure Tetranatriumsalz (Durchführung analog der Beispiele 1 und 2 der DE 4211713, wobei anstelle der Asparaginsäure das Mononatriumsalz der Glutaminsäure eingesetzt wurde)

In einem Reaktionsgefäß legte man einer Lösung von 187 g (1,0 mol) L-Glutaminsäure Mononatriumsalz Monohydrat in 300,0 g Wasser vor. Hierzu tropfte man unter Rühren innerhalb von 1 Stunde gleichzeitig 200 g (2,0 mol) einer 30 Gew.-%igen wässrigen Formaldehydlösung und 54,0 g (2,0 mol) Blausäure, wobei die Temperatur durch Kühlen bei 20 bis 25°C gehalten wurde. Nach beendeter Zugabe erhitzte man über einen Zeitraum von 1 Stunde auf 70°C. Die erhaltene Reaktionsmischung wurde dann innerhalb von 1 Stunde zu 408,0 g (3,06 mol) einer 30 Gew.-%igen Natronlauge bei 40°C zugetropft. Nach Beendigung der Zugabe erhitzte man auf 100°C, bis nach etwa 4 Stunden keine Ammoniakentwicklung mehr feststellbar war. Es wurden 1041 g einer Lösung erhalten, die laut HPLC-Analytik 312,4 g des L-Glutaminsäure-N,N-diessigsäure Tetranatriumsalzes enthielt und damit eine etwa 30 Gew.-%ige Lösung des Produkts darstellte. Bezogen auf eingesetztes Glutamat ergab sich eine Ausbeute von 89%. Der mittels HPLC bestimmte Anteil an NTA Trinatriumsalz in der Lösung betrug 0,35 Gew.-%.

## Patentansprüche

1. Verfahren zur Herstellung von Aminodicarbonsäure-N,N-diessigsäuren der allgemeinen Formel I, 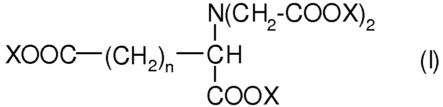 worin X unabhängig voneinander für Wasserstoff oder Alkalimetall steht und n eine Zahl 1 oder 2 bezeichnet,
umfassend die folgenden Schritte:
a) Umsetzung einer Aminodicarbonsäure der allgemeinen Formel II 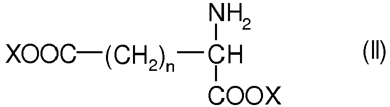 worin X und n die zuvor genannten Bedeutungen haben, mit 0,8 bis 1,2 Mol-Äquivalenten Formaldehyd und mit 0,8 bis 1,2 Mol-Äquivalenten Blausäure, umfassend die Zugabe von Formaldehyd und Blausäure, wobei man die Aminodicarbonsäure im Reaktionsgefäß vorlegt und Formaldehyd und Blausäure zugibt;
Nachreagieren lassen über einen Zeitraum von einer Minute bis 5 Stunden,
b) anschließende Umsetzung des in Schritt a) erhaltenen Reaktionsprodukts mit 0,8 bis 1,2 Mol-Äquivalenten Blausäure und mit 0,8 bis1,2 Mol-Äquivalenten Formaldehyd;
c) Hydrolyse des in Schritt b) erhaltenen Reaktionsprodukts.

2. Verfahren nach Anspruch 1, wobei man in Schritt a) zunächst den Formaldehyd und anschließend die Blausäure zu der Aminodicarbonsäure der allgemeinen Formel (II) gibt.

3. Verfahren nach einem der Ansprüche 1 oder 2, wobei in Schritt b) die Zugabe des Formaldehyd nach der Zugabe der Blausäure erfolgt.

4. Verfahren nach einem der Ansprüche 1 oder 2, wobei in Schritt b) die Umsetzung durch parallele Zugabe von Formaldehyd und Blausäure erfolgt

5. Verfahren nach einem der Ansprüche 1 bis 4, wobei die Umsetzung in Schritt a) in einem wässrigen Lösungsmittel durchgeführt wird.

6. Verfahren nach einem der Ansprüche 1 bis 5, wobei die Umsetzung in Schritt b) ohne vorherige Aufarbeitung des in Schritt a) erhaltenen Reaktionsprodukts erfolgt.

7. Verfahren nach einem der Ansprüche 1 bis 6, wobei die Hydrolyse in Schritt c) ohne vorherige Aufarbeitung des in Schritt b) erhaltenen Reaktionsprodukts erfolgt.

8. Verfahren nach einem der Ansprüche 1 bis 7, wobei die Hydrolyse in Schritt c) unter basischen Bedingungen erfolgt.

9. Verfahren nach Anspruch 8, wobei die Hydrolyse mit wässriger Natriumhydroxidlösung erfolgt.

10. Verfahren nach einem der Ansprüche 1 bis 9, wobei in Schritten a) und b) jeweils sowohl für Formaldehyd als auch für Blausäure 0,9 bis 1,1 Mol-Äquivalente eingesetzt werden.

11. Verfahren nach einem der Ansprüche 1 bis 10, wobei n = 2 ist.

12. Verfahren nach Anspruch 11, wobei in Schritt a) L-Glutaminsäure Mononatriumsalz als Verbindung II eingesetzt wird.

13. Verfahren nach einem der Ansprüche 1 bis 12, wobei das Verfahren kontinuierlich durchgeführt wird.

14. Verfahren nach einem der Ansprüche 1 bis 12, wobei das Verfahren diskontinuierlich durchgeführt wird.

15. Verfahren nach Anspruch 14, wobei das Verfahren im Semibatch-Verfahren durchgeführt wird.

## Claims

1. A process for preparing aminodicarboxylic acid-N,N-diacetic acids of the general formula I 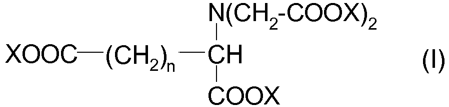 in which X is independently hydrogen or alkali metal and n is 1 or 2,
comprising the following steps:
a) reacting an aminodicarboxylic acid of the general formula II 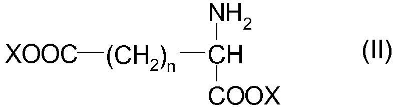 in which X and n are each as defined above with from 0.8 to 1.2 molar equivalents of formaldehyde and with from 0.8 to 1.2 molar equivalents of hydrocyanic acid, comprising the addition of formaldehyde and hydrocyanic acid, the aminodicarboxylic acid being initially charged in the reaction vessel and formaldehyde and hydrocyanic acid being added;
allowing the mixture to continue to react over a period of from one minute to 5 hours,
b) then reacting the reaction product obtained in step a) with from 0.8 to 1.2 molar equivalents of hydrocyanic acid and with from 0.8 to 1.2 molar equivalents of formaldehyde;
c) hydrolyzing the reaction product obtained in step b).

2. The process according to claim 1, wherein, in step a), first the formaldehyde and then the hydrocyanic acid is added to the aminodicarboxylic acid of the general formula (II).

3. The process according to either of claims 1 and 2, wherein, in step b), the formaldehyde is added after the hydrocyanic acid has been added.

4. The process according to either of claims 1 and 2, wherein, in step b), the reaction is effected by parallel addition of formaldehyde and hydrocyanic acid.

5. The process according to any one of claims 1 to 4, wherein the reaction in step a) is carried out in an aqueous solvent.

6. The process according to any one of claims 1 to 5, wherein the reaction in step b) is effected without preceding workup of the reaction product obtained in step a).

7. The process according to any one of claims 1 to 6, wherein the hydrolysis in step c) is effected without preceding workup of the reaction product obtained in step b).

8. The process according to any one of claims 1 to 7, wherein the hydrolysis in step c) is effected under basic conditions.

9. The process according to claim 8, wherein the hydrolysis is effected with aqueous sodium hydroxide solution.

10. The process according to any one of claims 1 to 9, wherein, in steps a) and b), from 0.9 to 1.1 molar equivalents are used for each of formaldehyde and hydrocyanic acid.

11. The process according to any one of claims 1 to 10, wherein n = 2.

12. The process according to claim 11, wherein, in step a), L-glutamic acid monosodium salt is used as the compound II.

13. The process according to any one of claims 1 to 12, which is performed continuously.

14. The process according to any one of claims 1 to 12, which is performed batchwise.

15. The process according to claim 14, which is performed semibatchwise.

## Revendications

1. Procédé de fabrication d'acides aminodicarboxylique-N,N-diacétiques de formule générale I 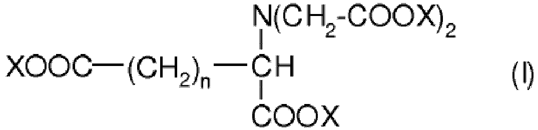 dans laquelle les X représentent indépendamment les uns des autres l'hydrogène ou un métal alcalin et n désigne un nombre 1 ou 2,
comprenant les étapes suivantes :
a) la mise en réaction d'un acide aminodicarboxylique de formule générale II 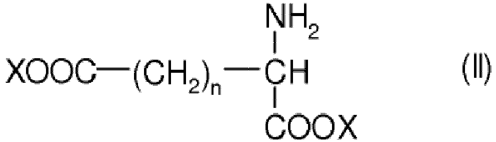 dans laquelle X et n ont les significations indiquées précédemment, avec 0,8 à 1,2 équivalent molaire de formaldéhyde et avec 0,8 à 1,2 équivalent molaire d'acide cyanhydrique, comprenant l'ajout de formaldéhyde et d'acide cyanhydrique, l'acide aminodicarboxylique étant chargé dans le récipient de réaction et le formaldéhyde et l'acide cyanhydrique étant ajoutés ;
et laissés réagir pendant une durée allant d'une minute à 5 heures, puis
b) la mise en réaction du produit de réaction obtenu à l'étape a) avec 0,8 à 1,2 équivalent molaire d'acide cyanhydrique et avec 0,8 à 1,2 équivalent molaire de formaldéhyde ;
c) l'hydrolyse du produit de réaction obtenu à l'étape b).

2. Procédé selon la revendication 1, dans lequel, à l'étape a), tout d'abord le formaldéhyde, puis l'acide cyanhydrique sont ajoutés à l'acide aminodicarboxylique de formule générale (II).

3. Procédé selon l'une quelconque des revendications 1 ou 2, dans lequel, à l'étape b), l'ajout du formaldéhyde a lieu après l'ajout de l'acide cyanhydrique.

4. Procédé selon l'une quelconque des revendications 1 ou 2, dans lequel, à l'étape b), la réaction a lieu par ajout parallèle de formaldéhyde et d'acide cyanhydrique.

5. Procédé selon l'une quelconque des revendications 1 à 4, dans lequel la réaction à l'étape a) est réalisée dans un solvant aqueux.

6. Procédé selon l'une quelconque des revendications 1 à 5, dans lequel la réaction à l'étape b) a lieu sans traitement préalable du produit de réaction obtenu à l'étape a).

7. Procédé selon l'une quelconque des revendications 1 à 6, dans lequel l'hydrolyse à l'étape c) a lieu sans traitement préalable du produit de réaction obtenu à l'étape b).

8. Procédé selon l'une quelconque des revendications 1 à 7, dans lequel l'hydrolyse à l'étape c) a lieu en conditions basiques.

9. Procédé selon la revendication 8, dans lequel l'hydrolyse a lieu avec une solution aqueuse d'hydroxyde de sodium.

10. Procédé selon l'une quelconque des revendications 1 à 9, dans lequel, aux étapes a) et b), 0,9 à 1,1 équivalent molaire sont utilisés aussi bien pour le formaldéhyde que pour l'acide cyanhydrique.

11. Procédé selon l'une quelconque des revendications 1 à 10, dans lequel n = 2.

12. Procédé selon la revendication 11, dans lequel, à l'étape a), le sel de monosodium de l'acide L-glutamique est utilisé en tant que composé II.

13. Procédé selon l'une quelconque des revendications 1 à 12, dans lequel le procédé est réalisé de manière continue.

14. Procédé selon l'une quelconque des revendications 1 à 12, dans lequel le procédé est réalisé de manière discontinue.

15. Procédé selon la revendication 14, dans lequel le procédé est réalisé de manière semi-discontinue.
